# EUROPEAN PATENT SPECIFICATION

(11) **EP 4 410 344 B1**
(45) Date of publication and mention of the grant of the patent: **17.06.2026**
(21) Application number: 22875201.0
(22) Date of filing: 30.09.2022
(51) Int. Cl.: A61M 16/06

(54) **ORONASAL PAD AND PATIENT INTERFACE DEVICE**
ORONASALES KISSEN UND PATIENTENSCHNITTSTELLENVORRICHTUNG
TAMPON ORONASAL ET DISPOSITIF D'INTERFACE PATIENT

(30) Priority: 30.09.2021 CN 202111165555
(43) Date of publication of application: 07.08.2024
(73) Proprietor: BMC (Tianjin) Medical Co., Ltd., Tianjin 301700 (CN)
(72) Inventor: WANG, Yajie, Tianjin 301700 (CN); ZHOU, Mingzhao, Tianjin 301700 (CN); ZHUANG, Zhi, Tianjin 301700 (CN)
(74) Representative: Grünecker Patent- und Rechtsanwälte PartG mbB
(86) International application number: PCT/CN2022/123530
(87) International publication number: WO 2023/051821

(56) References cited:
- EP-B1- 2 968 820
- WO-A1-2012/040792
- CN-A- 103 153 378
- CN-A- 112 041 013
- CN-A- 112 041 013
- CN-A- 114 010 902
- CN-U- 217 366 825
- US-A1- 2007 125 385
- US-A1- 2017 128 689
- US-A1- 2017 128 689
- US-A1- 2020 206 446

## Description

### CROSS-REFERENCE TO RELATED APPLICATIONS

This application claims priority to Chinese patent application CN 202111165555.8 filed on September 30, 2021.

### TECHNICAL FIELD

The present disclosure relates to the technical field of the treatment of respiratory-related diseases, and in particular to an oronasal cushion and a patient interface device.

### BACKGROUND

Respiratory-system-related diseases are accompanied by a series of breathing disorders, characterized by apnea, hypopnea and hyperpnea. Examples of respiratory diseases include obstructive sleep apnea (OSA), respiratory insufficiency, obesity, chronic obstructive pulmonary disease (COPD) and the like. A patient interface device, also known as a face mask, is generally used for the treatment of the above respiratory diseases.

As can be seen from currently known clinical data in combination with a general usage of devices, a full-face mask is an interface configuration that is most frequently used by a wearer. The full-face mask, as the name suggests, will simultaneously cover and seal oral features and nasal features of the wearer during the treatment, which results in some insurmountable defects of the full-face mask itself. For example, the full-face mask needs to cover the nose and mouth of the wearer when it is used, so the full-face mask will be bulky in the entirety, and it will seriously affect the visual field of the wearer when being used. Further, since the full-face mask covers the nose of the wearer, it will also press the sensitive nasal bridge region of the wearer, and is unfriendly to wearers who suffer from claustrophobia, and so on. All of these defects may lead to the resistance of the wearer. WO 2012/040792 A1 discloses a mask system for delivery of respiratory therapy to a patient comprising a nares portion and a mouth portion, wherein an inlet conduit connected to at least one of the nares portion and the mouth portion to deliver the pressurized, breathable gas. EP 2938820 B1 discloses a subnasal sealing cushion for a patient interface device comprising a cushion member having a front side and a rear side, wherein the cushion member includes a support portion having an outer wall and a support structure coupled to the outer wall and extending longitudinally along a first direction extending from a front side to a rear side, and a sealing flap portion including a nasal shelf portion defining a nasal orifice and being structured to contact a bottom of a nose of the user to create a seal therewith responsive to the patient interface device being donned by the user. US 2007/0125385 A1 discloses a patient interface that reliably and comfortably seals a user's face by providing an oral-nasal mask that includes an integrated nasal interface, comprising a seal member having an oral cushion and a nasal interface portion configured to surround the user's mouth and a nasal interface portion that provides an interface with the user's nose, wherein the oral cushion portion and the nasal interface portion is a unitary member, and the nasal interface portion remains below the bridge of the nose. CN 112041013 A discloses a plenum chamber for a patient interface comprising an oral portion, a nasal portion, a seal-forming structure, and a shell, wherein the seal-forming structure includes arrangements having areas with a higher resistance to deform at laterally spaced portions of either the nasal portion or the oral portion, and another seal-forming structure includes a bridge portion that can be deformed to accommodate different patient nose lengths, and a third seal-forming structure includes a different finish on the nasal portion and the oral portion. US 2020/0206446 A1 discloses an oro-nasal ventilation face mask worn by a patient includes a first chamber receiving airflow from an air source, a second chamber in fluid communication with the first chamber, a restrictive element positioned between the first chamber and the second chamber, and a headgear securing the first chamber and the second chamber to the patient.

### SUMMARY

The present disclosure provides an oronasal cushion and a patient interface device to solve the above technical problems. The invention is defined by the appended claims.

The present invention provides an oronasal cushion, including a nasal structure and an oral structure connected to each other, a penetrating cavity is formed inside the nasal structure and the oral structure, and the cavity is configured to receive pressurized gas; the nasal structure is adapted to fit around nostrils of a wearer for sealing, and the oral structure is adapted to fit around a mouth of the wearer for sealing;
wherein the nasal structure is configured so that no clamping effect is applied on ala nasi of the wearer when the nasal structure is pressed against a bottom of the nose of the wearer for sealing by the pressurized gas;
the oral structure is configured to accommodate the mouth of the wearer and fit around the mouth of the wearer in response to the oronasal cushion being put on by the wearer; and
the oral structure includes an oral soft pad part in contact with a face of the wearer and a reinforcing structure connected with the oral soft pad part, and rigidity of the reinforcing structure is greater than rigidity of the oral soft pad part;
wherein the nasal structure includes a nasal opening in communication with the cavity and a nasal soft pad part surrounding the nasal opening, the nasal opening is configured to surround a lower side of the nostrils of the wearer in response to the oronasal cushion being put on by the wearer, and the nasal soft pad part is adapted to fit around the nostrils of the wearer for sealing;
wherein the nasal soft pad part includes a soft intermediate part disposed around the nasal opening, the intermediate part is configured to accommodate the nose of the wearer without submerging a nasal apex of the wearer, and the intermediate part deforms in response to an increase in the pressure in the cavity, to fit around the nostrils of the wearer'.

In an embodiment, the nasal soft pad part further includes a first lateral support part and a second lateral support part connected to the intermediate part respectively;
wherein the first lateral support part and the second lateral support part extend on both sides of the intermediate part, respectively; or
the first lateral support part and the second lateral support part each extend on both sides of the intermediate part respectively until reaching an outer front end of the intermediate part.

In an embodiment, the intermediate part is configured to be further away from an upper end of the oronasal cushion than the first lateral support part and the second lateral support part.

In an embodiment, rigidity of both the first lateral support part and the second lateral support part is greater than rigidity of the intermediate part.

In an embodiment, a thickness of the first lateral support part and a thickness of the second lateral support part are greater than a thickness of the intermediate part.

In an embodiment, the intermediate part is a structure with uniform thickness.

In an embodiment, a thickness of the intermediate part is 0.2mm~1.0mm.

In an embodiment, the intermediate part includes one or more local thickened part.

In an embodiment, the local thickened part is provided at a peripheral edge of the intermediate part.

In an embodiment, a thickness of the local thickened part is 0.2mm~1.2mm.

In an embodiment, a thickness of the first lateral support part and a thickness of the second lateral support part are both 0.6mm~1.5mm.

In an embodiment, the oronasal cushion also includes an oronasal transition part disposed between a rear side of the intermediate part and the oral structure, the oronasal transition part is configured to fit with an upper lip region of the wearer in response to the oronasal cushion being put on by the wearer; and
a thickness of the oronasal transition part is the same as a thickness of the intermediate part.

In an embodiment, the oronasal transition part is configured as a concave structure recessed in a direction away from upper lip of the wearer.

In an embodiment, the thickness of the oronasal transition part is the same as the thickness of the intermediate part.

In an embodiment, two sides of the oronasal transition part are respectively connected to the first lateral support part and the second lateral support part by recessing in a direction away from upper lip of the wearer.

In an embodiment, the oronasal cushion also includes an oronasal connection part provided between the nasal structure and the oral structure, and a thickness of the oronasal connection part is variable.

In an embodiment, the oronasal connection part includes a first oronasal connection part located between the first lateral support part and a side part of the oral structure, a second oronasal connection part located between the second lateral part and the side part of the oral structure and a third oronasal connection part located between a front side of the nasal structure and a front side of the oral structure; and
a thickness of the first oronasal connection part and a thickness of the second oronasal connection part are both greater than a thickness of the third oronasal connection part.

In an embodiment, the thickness of the oronasal connection part is 0.2mm~0.6mm.

In an embodiment, the oral structure further includes an oral opening in communication with the cavity, the oral soft pad part is disposed around the oral opening, and the oral opening is configured to accommodate the mouth of the wearer in response to the oronasal cushion being put on by the wearer;
a region of the oral structure where the oral opening is located is recessed inward the cavity, and a region of the oral structure where the oral soft pad part is located protrudes outward the cavity.

In an embodiment, the oral opening is configured in an elliptical or quasi-elliptical shape.

In an embodiment, the oral soft pad part includes an oral ambilateral part and a chin part, and an oronasal transition part and the chin part are respectively connected to an upper side and a lower side of the oral ambilateral part; the oral ambilateral part is configured to fit with the face of the wearer in response to the oronasal cushion being put on by the wearer, and the chin part is configured to fit with chin of the wearer in response to the oronasal cushion being put on by the wearer.

In an embodiment, the oral ambilateral part includes a face contact region surrounding the oral opening, a face support region smoothly connected to the face contact region and extending toward a front side of the oral structure, and an oral transition region surrounding the oral opening; the oral transition region is smoothly connected to the face contact region and the face support region.

In an embodiment, a thickness of the oral transition region is less than a thickness of the face support region.

In an embodiment, a thickness of the face contact region is less than or equal to a thickness of the face support region.

In an embodiment, a thickness of the face support region is 1.2mm~2.5mm.

In an embodiment, a thickness of the oral transition region is 0.3mm~0.6mm.

In an embodiment, the chin part includes a chin contact region surrounding the oral opening and a chin transition region smoothly connected to the chin contact region, the chin transition region extends to a front side of the oronasal cushion, or the chin transition region is connected with the chin support region.

In an embodiment, a thickness of the chin transition region is the same as a thickness of the chin contact region.

In an embodiment, a thickness of the chin transition region is 0.2mm~0.8mm.

In an embodiment, the reinforcing structure is located on a front side of the oral structure and is connected to the oral soft pad part of the oral structure, the reinforcing structure is provided with an air inlet in communication with the cavity, the air inlet is provided with a sealing part, and the air inlet is sealingly connected to a frame of a patient interface device through the sealing part, and the pressured gas is allowed to be supplied into the cavity through the air inlet.

In an embodiment, the reinforcing structure and the oral structure are integrally formed from the same material; or
the reinforcing structure and the oral structure are made from different materials respectively, and are connected through a connection part on the reinforcing structure.

In an embodiment, a part between the air inlet of the reinforcement structure and the connection part on the reinforcement structure is a support transition region, and a thickness of the support transition region is 0.8mm~2.5mm.

In an embodiment, the reinforcing structure is made from Polycarbonate (PC), Polypropylene (PP), acrylic and Acrylonitrile Butadiene Styrene (ABS).

In an embodiment, the nasal structure and the oral structure are made from the same or different materials.

In an embodiment, the nasal structure is made from one or more of silicone rubber, foam, thermoplastic elastomer, thermosetting material, resin and textile.

In an embodiment, the oral structure is made from one or more of silicone rubber, foam, thermoplastic elastomer, thermosetting material, resin and textile.

According to a second aspect of the present disclosure, the present disclosure provides an oronasal cushion, including a nasal structure and an oral structure connected to each other; the nasal structure is adapted to fit around nostrils of a wearer for sealing; wherein the nasal structure is configured so that no clamping effect is applied on ala nasi of the wearer when the nasal structure is pressed against the bottom of the wearer's nose for sealing by the pressurized gas; the oral structure is configured to accommodate a mouth of the wearer and fit around the mouth of the wearer in response to the oronasal cushion being put on by the wearer;
wherein the oronasal cushion also includes exhaust components and/or ventilation components; the exhaust components are configured to discharge the gas exhaled by the wearer in the oronasal cushion, and the ventilation components are configured to ensure the oronasal cushion to be communicated with the environment when no gas is supplied into the oronasal cushion.

In an embodiment, the oronasal cushion further includes a reinforcing structure located on a front side of the oral structure and connected to an oral soft pad part of the oral structure, and an air inlet is provided on the reinforcing structure, the exhaust components are arranged on both sides of the air inlet, respectively.

In an embodiment, the oronasal cushion further includes a reinforcing structure located on a front side of the oral structure and connected to an oral soft pad part of the oral structure, and an air inlet is provided on the reinforcing structure, the ventilation components are arranged on both sides of the air inlet, respectively.

In an embodiment, the oronasal cushion further includes a reinforcing structure located on a front side of the oral structure and connected to an oral soft pad part of the oral structure, and an air inlet is provided on the reinforcing structure, the exhaust component and the ventilation component are arranged on both sides of the air inlet, respectively.

In an embodiment, the exhaust component comprises exhaust holes, and an inner hole diameter of each of the exhaust holes is less or greater than an outer hole diameter of each of the exhaust holes.

In an embodiment, the exhaust holes are in a shape of a strip, an oblong or a circle.

In an embodiment, the exhaust component comprises a plurality of exhaust holes that are arranged in a circular arrangement, an elliptical arrangement, or an array hole arrangement.

In an embodiment, the exhaust holes are arranged in a divergent manner, and a number of adjacent exhaust holes for each exhaust hole is at most 6.

In an embodiment, the exhaust holes are provided on both sides of the air inlet and far away from the air inlet.

In an embodiment, the exhaust holes are formed by kissing off on the mold.

In an embodiment, the ventilation component comprises a safety valve port and a safety valve disc;
when no gas is supplied into the oronasal cushion, the safety valve disc opens the safety valve port, to allow the oronasal cushion to communicate with the environment; when gas is supplied into the oronasal cushion, the safety valve disc closes the safety valve port.

According to a third aspect of the present disclosure, the present disclosure also provides a patient interface device, including the oronasal cushion as described above and a frame, the oronasal cushion is sealingly connected to the frame.

Compared with the related art, the advantages of the present disclosure are as follows: since the nasal opening in the nasal structure surrounds the lower side of the nostrils of the wearer for ventilation, the oronasal cushion of the present disclosure does not need to cover the nose of the wearer, thereby reducing its overall volume and making it more compact and lightweight. Thus, it is less likely to cause claustrophobia and makes it more comfortable to wear. Moreover, the nasal soft pad part adaptively fits around the nostrils of the wearer for sealing, so the ala nasi of the wearer will not be clamped, thereby further improving the wearing comfort, and improving treatment effectiveness and treatment compliance of the wearer. In addition, by constructing the nasal structure and the oral structure separately, the internal cavity can withstand higher pressure to adapt to the treatment needs of different symptoms.

### BRIEF DESCRIPTION OF THE DRAWINGS

The present disclosure will be described in detail below based on embodiments and with reference to the accompanying drawings.
FIG.1 is a schematic diagram illustrating a basic structure of a human nose;
FIG. 2 is a schematic diagram of a three-dimensional structure of an oronasal cushion according to Embodiment 1 of the present disclosure (viewed from a rear side);
FIG. 3 is a schematic diagram of a three-dimensional structure of the oronasal cushion according to Embodiment 1 of the present disclosure (viewed from a front side);
FIG. 4 is a front view of the oronasal cushion according to Embodiment 1 of the present disclosure;
FIG. 5a is a schematic diagram illustrating a positional relationship between the oronasal cushion and the nose of the wearer according to Embodiment 1 of the present disclosure, in which a thick dotted line indicates a position of an intermediate part when being deformed, and a thin dotted line indicates a dividing line between the intermediate part and nasal ambilateral part;
FIG. 5b is a schematic diagram illustrating a wearing condition of the oronasal cushion according to Embodiment 1 of the present disclosure, where the intermediate part has been expanded and deformed to stick close to the nostrils of the wearer, and the thin dotted line in the figure indicates the position of the intermediate part when it is not deformed;
FIG. 6a is a top view of the oronasal cushion according to Embodiment 1 of the present disclosure, in which the dotted line indicates a dividing line between the intermediate part and the nasal ambilateral part;
FIG. 6b is a cross-sectional view at C-C in FIG. 6a;
FIG. 6c is a cross-sectional view at D-D in FIG. 6a;
FIG. 7 is a cross-sectional view of the oronasal cushion according to Embodiment 1 of the present disclosure, in which the dotted line indicates the nose of the wearer;
FIG. 8 is a top view of an oronasal cushion according to another embodiment of the present disclosure, in which the dotted line indicates a dividing line between the intermediate part and the nasal ambilateral part;
FIG. 9 is a front view of the oronasal cushion according to Embodiment 1 of the present disclosure, in which a chine part is shown, and the dotted line in the figure indicates a dividing line between the oral ambilateral part and the chin part;
FIG. 10 is a cross-sectional view at A-A in FIG. 9;
FIG. 11 is a cross-sectional view at B-B in FIG. 9;
FIG. 12 is a schematic diagram of a three-dimensional structure of an oronasal cushion according to Embodiment 2 of the present disclosure (viewed from a rear side);
FIG. 13 is a schematic diagram of a three-dimensional structure of the oronasal cushion according to Embodiment 2 of the present disclosure (viewed from a front side);
FIG. 14 is a front view (viewed from the rear side) of the oronasal cushion according to Embodiment 2 of the present disclosure;
FIG. 15 is a cross-sectional view at E-E in FIG. 14;
FIG. 16 is a schematic diagram of a three-dimensional structure of an oronasal cushion according to Embodiment 3 of the present disclosure (viewed from a front side);
FIG. 17 is a schematic diagram of a three-dimensional structure of an oronasal cushion according to Embodiment 4 of the present disclosure (viewed from a front side); and
FIG. 18 is a schematic diagram of a three-dimensional structure of an oronasal cushion according to Embodiment 5 of the present disclosure (viewed from a front side).

### Reference signs:

1-oronasal cushion;
2-nasal structure; 21-nasal opening; 22-nasal soft pad part; 23-intermediate part; 24-nasal ambilateral part; 25-local thickened part;
3-oral structure; 31-oral opening; 32-oral soft pad part;
34-oral ambilateral part; 341-face contact region; 342-face support region; 343-oral transition region;
35-chin part; 351-chin contact region; 352-chin support region; 353-chin transition region;
4-reinforcing structure; 41-air inlet; 42-support transition region; 43-connection part; 411-sealing part;
5- oronasal transition part; 6-cavity; 60-exhaust component; 61-exhaust hole;
501-safety valve port; 502-safety valve disc;
N1-nostrils; N2-ala nasi; N3- nasal apex; N4-nasal bridge; N5-nasal depression;
F-cheek.

### DETAILED DESCRIPTION OF THE EMBODIMENTS

The present disclosure will be further described below in conjunction with the accompanying drawings.

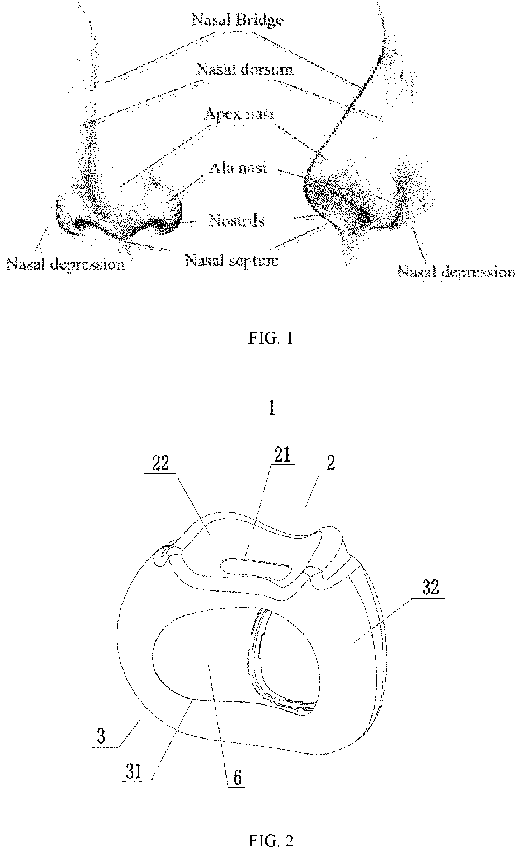

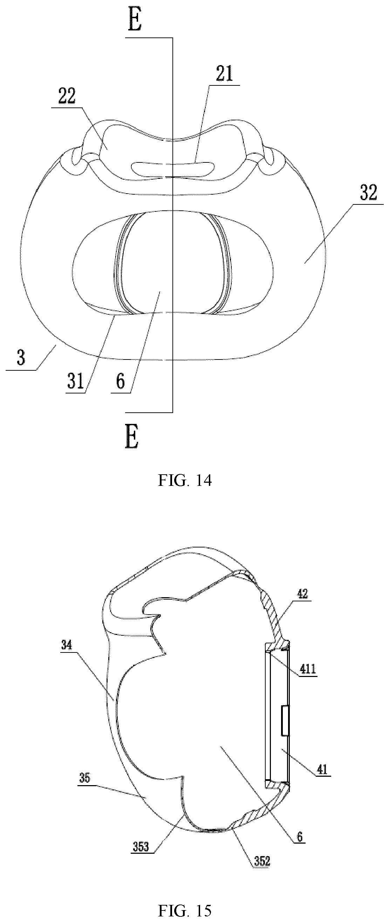

As shown in FIG. 1, nose features of a typical wearer are shown. As shown in FIG. 2 to FIG. 18, in a first aspect of the present disclosure, the present disclosure provides an oronasal cushion 1 that is arranged around a lower side of the nostrils of the wearer when being put on.

The oronasal cushion 1 of the present disclosure includes a nasal structure 2 and an oral structure 3 connected to each other. A penetrating cavity 6 is formed inside the nasal structure 2 and the oral structure 3. The cavity 6 may receive pressurized gas from a pressure device, and deliver the pressurized gas to the nose and mouth of the wearer through the nasal structure 2 and the oral structure 3 respectively. Therefore, the cavity 6 has an applicable pressure range of 4~40 hpa when it receives the pressurized gas, so it is necessary to provide the oral structure 3 to accommodate the mouth of the wearer and share part functions of the face contact and support.

Hereinafter, specific embodiments are used to describe the oronasal cushion 1 of the present disclosure.

### Embodiment 1

Referring to FIG. 2, the nasal structure 2 includes a nasal opening 21 connected with the cavity 6 and a nasal soft pad part 22 surrounding the nasal opening 21. The nasal opening 21 is configured to surround the lower side of the nostrils of the wearer in response to the oronasal cushion 1 being put on by the wearer, and the nasal soft pad part 22 is configured to fit around the nostrils of the wearer for sealing.

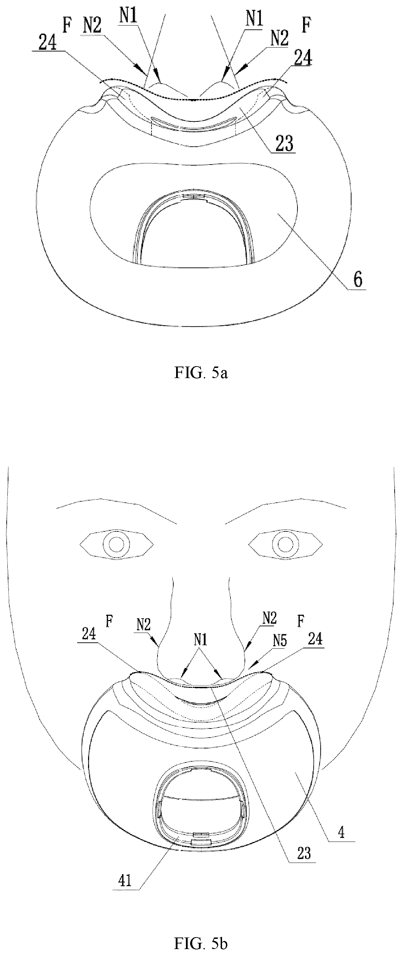

Specifically, referring to FIG. 5, the nasal soft pad part 22 includes an intermediate part 23 provided around the nasal opening 21 as well as a first lateral support part and a second lateral support part provided on two sides of the intermediate part 23. Since the first lateral support part and the second lateral support part may be constructed according to the same structure, hereinafter, the first lateral support part and the second lateral support part will no longer be distinguished, but are collectively referred to as the nasal ambilateral part 24. It should be understood that the first lateral support part and the second lateral support part may also be different in terms of thicknesses, materials or the like.

The intermediate part 23 deforms in response to an increase of the pressure within the cavity 6 to fit around the nostrils of the wearer. The thickness of the intermediate part 23 may be, for example, 0.2-1.0 mm, that is, the region where the intermediate part 23 is located is a thin film region. Therefore, when being under pressure, the intermediate part 23 will expand and deform so that it can fit closely around the nostrils of the wearer. Accordingly, although there are individual differences in the nose of the wearer, it is ensured that the intermediate part 23 can fit around the nostrils of different wearers due to the deformability of the intermediate part 23, thereby improving the scope of application and sealing stability of the intermediate part 23. In other words, since the oronasal cushion 1 of the present disclosure fits around the nostrils of the wearer through the expansion and deformation of the intermediate part 23, there is no need to consider nose differences of the wearers during designing and manufacturing. In other words, the nasal structure 2 can fit around the nostrils of the user for sealing adaptively, and the nasal structure 2 is configured such that the nasal structure 2 has no clamping effect against the alae nasi of the patient when it leans against the bottom of the nose of the wearer due to the pressurized gas for sealing. Therefore, even if there are differences in the noses of the wearers, the deformability of the film can ensure that the intermediate part 23 fits around the nostrils of the wearer adaptively, thereby improving the tightness of the contact between the oronasal cushion 1 and the face of the wearer and connection stability.

More specifically, the intermediate part 23 is recessed inward the cavity 6, that is, the intermediate part 23 is slightly lower than the nasal ambilateral part 24 (as shown in FIG. 5a). Therefore, the height of the intermediate part 23 will approach to the height of the nasal ambilateral part 24 when the intermediate part 23 is expanded by the pressure in the cavity 6, as shown in FIG.5b. When the intermediate part 23 is expanded, it changes from an original small arc circle to a large arc circle, and the intermediate part 23 and the nasal ambilateral part 24 can even be located in almost the same plane, thus ensuring that the part of the oronasal cushion 1 that is in contact with the face of the wearer is under the nose of the wearer to horizontally hold the nose, rather than wrapping around the alae nasi of the wearer. Therefore, the oronasal cushion 1 of the present disclosure can ensure the sealing stability without clamping the alae nasi of the wearer, so that the oronasal cushion 1 does not require to be strictly adapted to the width of the nose of the wearer (the distance between the alae nasi on both sides, as shown in FIG. 1), thereby increasing its adaptability to the population.

As shown in FIG. 5a, the intermediate part 23 is slightly lower than the nasal ambilateral part 24 when being not deformed, and can be located at the position shown by the thick dotted line in FIG. 5a when being expanded and deformed. As can be seen more clearly in FIG. 5b, the intermediate part 23 upward after deformation, so as to seal around the nostrils of the wearer N1, without covering the ala nasi N2 and the nasal depression N5 of the wearer.

Moreover, since the oronasal cushion 1 of the present disclosure does not clamp the ala nasi of the wearer, the contact region between the oronasal cushion and the ala nasi of the wearers can be reduced, and the wearing comfort can be improved.

Preferably, the front end 25 of the intermediate part 23 will not exceed the nasal apex N3 of the wearer, so that the nasal bridge N4 of the wearer will not be pressed when the oronasal cushion is put on, which can further improve the wearing comfort and also provide the wearer with a better field of view when the oronasal cushion is put on.

The nasal ambilateral part 24 are mainly used for supporting, so the stress thereof is greater than that of the intermediate part 23. Therefore, the thickness of the nasal ambilateral part 24 can be set to be greater than the thickness of the intermediate part 23. For example, the thickness of the nasal ambilateral part 24 may be 0.6-1.5 mm, preferably 0.8-1.2 mm, so that the rigidity of the nasal ambilateral part 24 may be greater than that of the intermediate part 23.

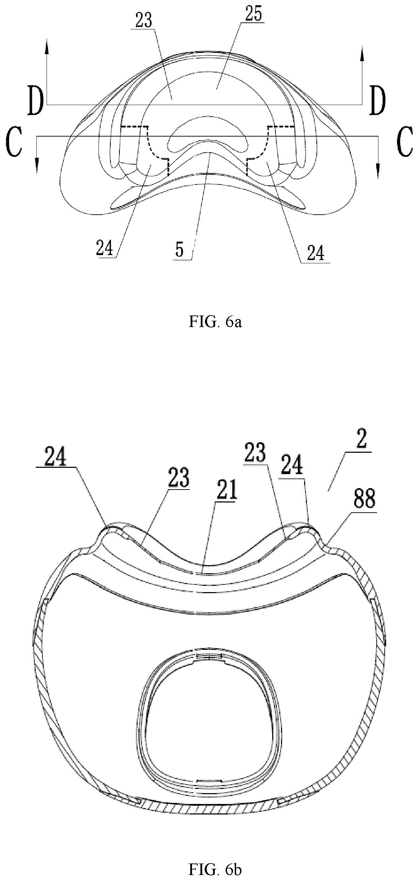

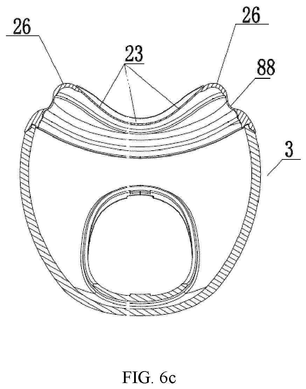

In an optional embodiment, as shown in FIG. 6a, FIG. 6b and FIG. 6c, the nasal ambilateral part 24 extend on both sides of the intermediate part 23 respectively. The dotted line in FIG. 6a shows the dividing line between the nasal ambilateral part 24 and the intermediate part 23, that is, the regions of the nasal ambilateral part 24 only extend in a small local region close to the face wearing side. In this embodiment, when the oronasal cushion 1 is worn, the nasal ambilateral part 24 first contact the face of the wearer and are closer to muscles of the cheek F of the wearer (as shown in FIG. 5a and FIG. 5b). Therefore, the wearer does not feel the force exerted on the nasal ambilateral part 2 significantly, so the facial pressure when the oronasal cushion 1 is worn can be effectively distributed by the nasal ambilateral part 24, thereby improving the wearing comfort. In addition, since the nasal ambilateral part 24 are close to the face of the wearer, in this case, the sealing stability of the oronasal cushion 1 at the nose depends on the supporting force of the nasal ambilateral part 24 and the expansion force of the intermediate part 23. In this embodiment, the region of the ambilateral part 24 only extends in a small local region close to the face wearing side.

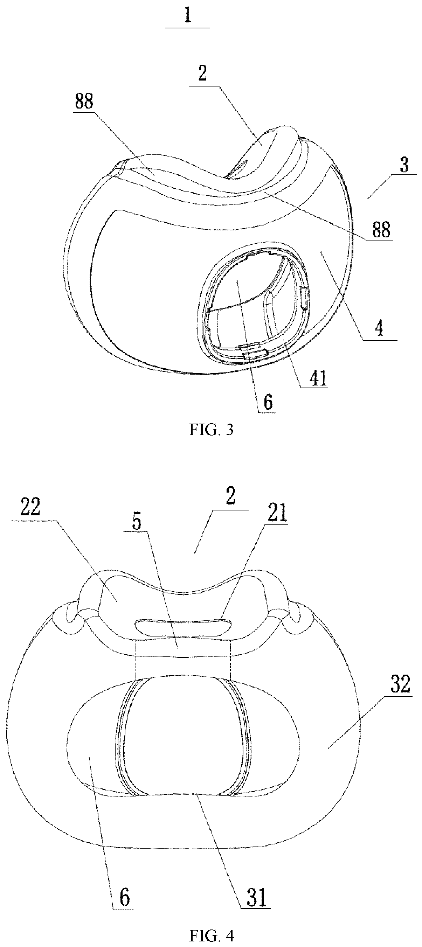

In this embodiment, as shown in FIG. 3, FIG. 6b, FIG. 6c and FIG. 7, an oronasal connection part 88 is provided between two sides of the nasal structure 2 and the oral structure 3 and between the front sides of the nasal structure 2 and the oral structure 3 respectively. That is to say, the oronasal connection part 88 includes: a first oronasal connection part located between the above-mentioned first lateral support part and the side of the oral structure 3; a second oronasal connection part located between the above-mentioned second lateral support part and the side of the oral structure 3; and a third oronasal connection part located between the front side of the nasal structure 2 (specifically the intermediate partial region 26 described below) and the front side of the oral structure 3. The oronasal connection part 88 has a variable thickness. Specifically, the thicknesses of the first oronasal connection part and the second oronasal connection part that are between the nasal ambilateral part 24 and the oral structure 3 is generally equal to or greater than the thickness of the third oronasal connection part of the nasal ambilateral part 24.

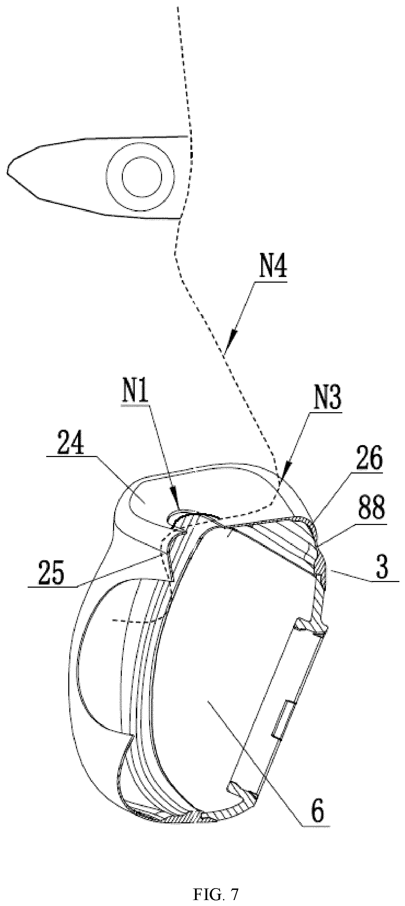

As shown in FIG. 6b and FIG. 7, the intermediate part 23 includes an intermediate partial region 26. The intermediate partial region 26 is basically located at an edge region of the nasal structure 2 that is not easily accessible by the wearer's nose. In this embodiment, the thickness of the intermediate partial region 26 is slightly greater than that of the intermediate part 23, for example 0.2mm~1.2mm, preferably 0.8-0.9mm, and the intermediate partial region 26 is mainly used for maintaining a rough shape of the film region of the intermediate part 23 when it is not inflated.

In addition, the intermediate partial region 26 may also have the same thickness as the intermediate part 23 to ensure that there is no major impact on use.

Referring to FIG. 6b and FIG.7, the transition thickness of the oronasal connection part 88 between the oral structure 3 and the intermediate part 23 of the nasal structure 2 may also be set as a film thickness. The thickness of the oronasal connection part 88 may be set as 0.2-0.6mm, preferably 0.3mm~0.5mm, so that the oronasal connection part 88 can better supplement the sealing at the nose region when it is inflated.

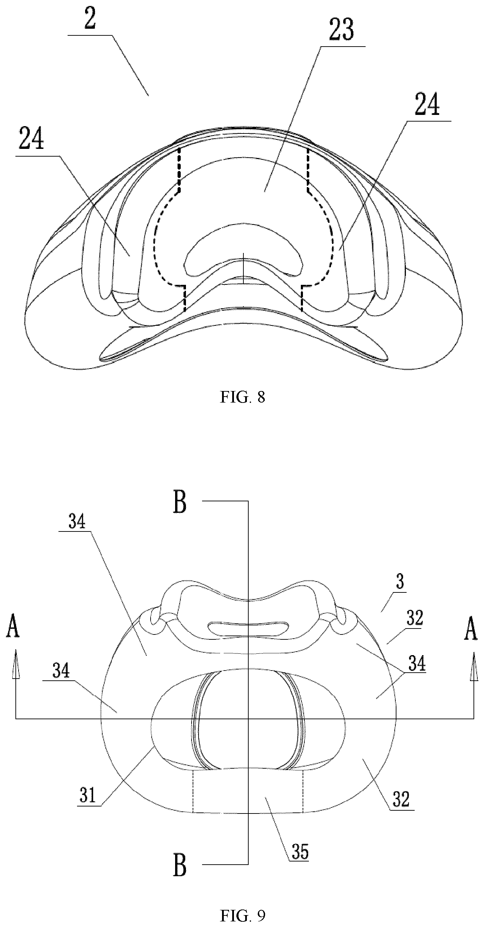

In another optional embodiment, the nasal ambilateral part 24 extends on both sides of the intermediate part 23 until reaching an outer front end of the intermediate part 23, so as to increase the area of the support region, as shown in FIG. 8, the dotted line indicates the dividing line between the nasal ambilateral part 24 and the intermediate part 23. In this embodiment, the area of the nasal ambilateral part 24 is larger, which can provide better supporting. Therefore, in this case, the sealing stability of the oronasal cushion 1 at the nose mainly depends on the supporting forces of the nasal ambilateral part 24. In this embodiment, the region of the ambilateral part 24 extends to the front side in a large range.

As stated above, the intermediate part 23 has a thin thickness and is a thin film region. Accordingly, optionally, the intermediate part 23 may be configured as a structure with a uniform thickness. Optionally, the intermediate part 23 further has one or more local thickened parts 25 to thicken local regions.

The number of nasal openings 21 may be set as needed. In this embodiment, one nasal opening 21 is shown, and multiple nasal openings 21 may also be provided as needed. For example, two nasal openings 21 are provided, and the nasal openings correspond to two nostrils of the wearer respectively, and surround the lower side of the wearer's corresponding nostrils when the oronasal cushion is worn.

The oronasal cushion 1 also includes an oronasal transition part 5 disposed between the nasal structure 2 and the oral structure 3. The oronasal transition part 5 and the upper lip region of the wearer have a close fit in response to the oronasal cushion 1 being put on by the wearer. The oronasal transition part 5 is configured as a concave shape in a direction away from the upper lip. Since the oronasal transition part 5 corresponds to a stress-sensitive region on the face of the wearer, the thickness of the oronasal transition part 5 may be set to be the same as or similar to the thickness of the intermediate part 23, that is, the region where the oronasal transition part 5 is located is also a film region, so as to ensure that the force on the upper lip region of the wearer is small enough when the oronasal cushion is worn, thereby improving the wearing comfort.

In the embodiment shown in FIG. 5a and FIG. 5b, a transition trend from the nasal ambilateral part 24 to the oronasal transition part 5 may be a concave shape in a direction away from the upper lip, so that the nasal ambilateral part 24 can contact the vicinity of the nasal depression N5 on the face first when the oronasal cushion is worn. Therefore, the nasal ambilateral part 24 is an important supporting point for the face F when a mask is worn. The force on the nasal ambilateral part 24 is greater than that on the sensitive intermediate part 23 of the nose and the oronasal transition part 5. On the other hand, since the nasal ambilateral part 24 supports the vicinity of the nasal depression N5, and is closer to the muscle of the cheek F (as shown in FIG. 5b), so the perception of force is not obvious, which can effectively distribute the pressure of wearing the pad on the face and improve the wearing comfort.

The oral structure 3 will be described in detail below.

Referring to FIG. 4, the oral structure 3 includes an oral opening 31 in communication with the cavity and an oral soft pad part 32 provided around the oral opening 31. The oral opening 31 is configured to accommodate the mouth of the wearer in response to the oronasal cushion 1 being put on by the wearer. The oral soft pad part 32 is configured to fit with the face of the wearer in response to the oronasal cushion 1 being put on by the wearer. Therefore, the oral structure 3 as a whole presents a tendency that the middle part where the oral opening 31 is located is recessed inward the cavity 6, while the ambilateral part where the oral soft pad part 32 is located protrudes outside, thereby ensuring a larger contact area between the oral soft pad part 32 and the face of the wearer to ensure the sealing effect.

The cross section of the oral opening 31 in a radial direction is quasi-elliptical or elliptical, and when the cushion is worn, the oral soft pad part 32 seals the mouth of the wearer.

As shown in FIG. 9, the oral soft pad part 32 includes an oral ambilateral part 34 and a chin part 35, and the dotted line indicates the dividing line between the oral ambilateral part 34 and the chin part 35. The oronasal transition part 5 and the chin part 35 are connected to the upper and lower sides of the oral ambilateral part 34, respectively; the oral ambilateral part 34 is configured to fit with the face of the wearer in response to the oronasal cushion 1 being put on by the wearer, and the chin part 35 is configured to fit with the chin of the wearer in response to the oronasal cushion 1 being put on by the wearer.

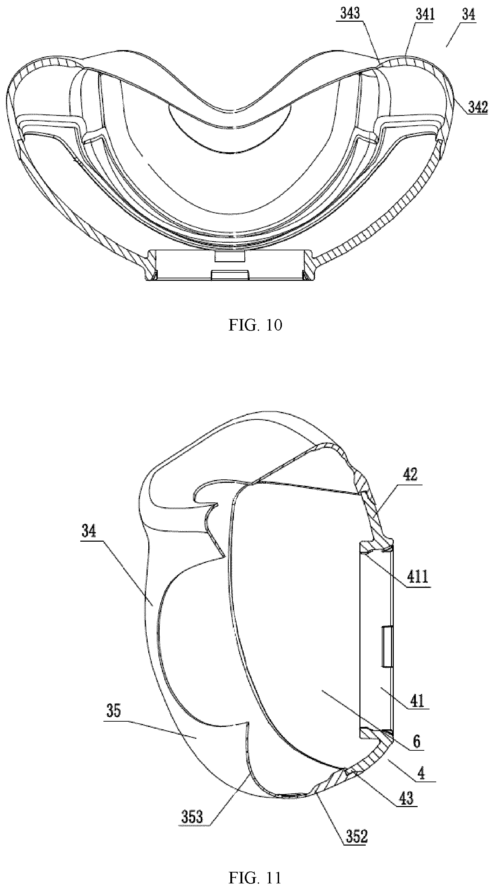

As shown in FIG. 10, the oral ambilateral part 34 includes: a face contact region 341 surrounding the oral opening 31; a face support region 342 that is smoothly connected to the face contact region 341 and extends toward the front side of the oral structure 3; and an oral transition region 343 surrounding the oral opening 31, which is smoothly connected to the face contact region 341 and the face support region 342.

Preferably, the thickness of the oral transition region 343 is less than the thickness of the face support region 342, and the thickness of the face contact region 341 is less than or equal to the thickness of the face support region 342. When the oronasal cushion 1 is worn, the face support region 342 is the main force-bearing support place to ensure the sealing stability with the wearer's face. Therefore, the face support region 342 needs to have a certain rigidity, then the thickness thereof may be 1.2-2.5mm, preferably about 1.5mm.

In addition, the face contact region 341 is close to the face of the wearer, thus it may have the same thickness as the face support region 342. The oral transition region 343 may have a smaller thickness, for example 0.3-0.6mm, and the region where the oral transition region 343 is located may also be a film region. Alternatively, the oral transition region 343 may also adopt other thicknesses less than the face support region 342.

The chin part 35 includes a chin contact region 351 surrounding the oral opening 31, a chin transition region 353 smoothly connected to the chin contact region 351, and a chin support region 352 that is smoothly transited and connected to the chin transition region 353. The area of the chin support region 352 may be very small, and even approaches to zero. At this time, the area of the chin transition region 353 is larger, and the chin transition region 353 may directly extend to the front side of the oronasal cushion 1.

The chin contact region 351 may have a smaller thickness, forming a thin film region. For example, the thickness of the chin contact region may be 0.2-0.8mm, preferably 0.2~0.5mm.

Since the chin transition region 353 corresponds to the chin (jaw) of the wearer, which is also a force-sensitive part, the thickness d4 of the chin transition region 353 is the same as or approximate to the thickness d5 of the chin contact region 351, thereby reducing the force exerted on the chin of the wearer when the oronasal cushion is worn, and improving the wearing comfort.

In this embodiment, the oronasal cushion 1 also includes a reinforcing structure 4 that is located on the front side of the oral structure 3 and connected to the oral soft pad part 32 of the oral structure 3. The reinforcing structure 4 is provided with an air inlet 41 in communication with the cavity, and the air inlet 41 is provided with a sealing part 411. The air inlet 41 is sealingly connected to the frame of the patient interface device through the sealing part 411. The pressurized gas may be supplied into the cavity through the air inlet 41.

Optionally, the reinforcing structure 4 and the oral structure 3 are integrally formed through injection molding.

Optionally, the reinforcing structure 4 and the oral structure 3 are formed from different materials respectively, and they are connected through the connecting part 43 on the reinforcing structure 4. The connection part 43 may be a mechanical connection component or a chemical adhesive layer.

The part between the air inlet 41 and the connection part 43 of the reinforcing structure 4 is a supporting transition region 42. Generally, the thickness of the supporting transition region 42 may be between 0.8-2.5 mm, preferably between 1.2-1.8 mm. In this way, not only the strength of the reinforcing structure 4 but also the portability thereof can be ensured.

The reinforcing structure 4 may be made from plastic materials such as Polycarbonate (PC) or Polypropylene (PP), or other thermoplastic materials such as highly transparent acrylic and Acrylonitrile Butadiene Styrene (ABS). Preferably, the reinforcing structure 4 is made from transparent PC.

Both the nasal structure 2 and the oral structure 3 may be made from silicone rubber, or one or more of foam, thermoplastic elastomer, thermosetting material, foam, resin, textile and other materials.

When both the nasal structure 2 and the oral structure 3 are made from silicone rubber, the silicone rubber with a Shore hardness of 30~40 is preferred.

### Embodiment 2

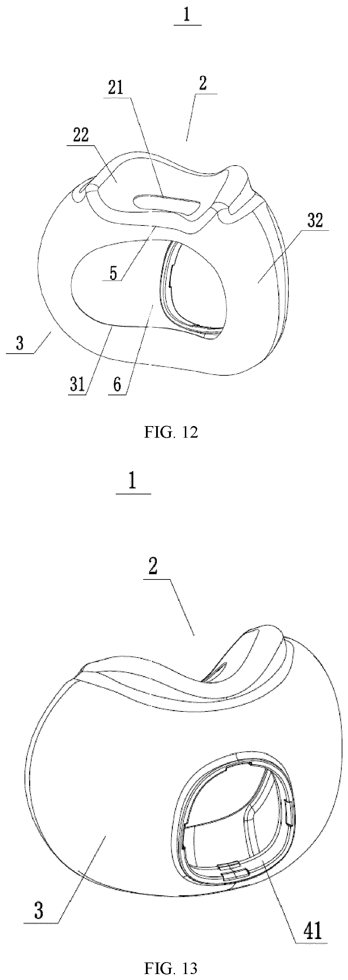

Based on Embodiment 1 described above, the present disclosure provides a modified embodiment, that is, Embodiment 2, as shown in FIG.12 to FIG.15.

The difference between this embodiment and Embodiment 1 lies in that the reinforcing structure 4 and the oral structure 3 are made from the same material, and form an integral body. The material of the nasal structure 2 may also be the same as the material of the reinforcing structure 4 and the oral structure 3, that is, the entire oronasal cushion 1 is made from the same material.

For example, the material of the oronasal cushion 1 may be one or more of silicone rubber, foam, thermoplastic elastomer, thermosetting material, foam, resin, textile and other materials.

When the material of the oronasal cushion 1 is silicone rubber, silicone rubber with a Shore hardness of 30~40 is preferred.

### Embodiment 3

Based on the above embodiments, the present disclosure provides a modified embodiment, that is, Embodiment 3. Only the differences from the previous embodiments will be described below, and the similarities will not be repeated.

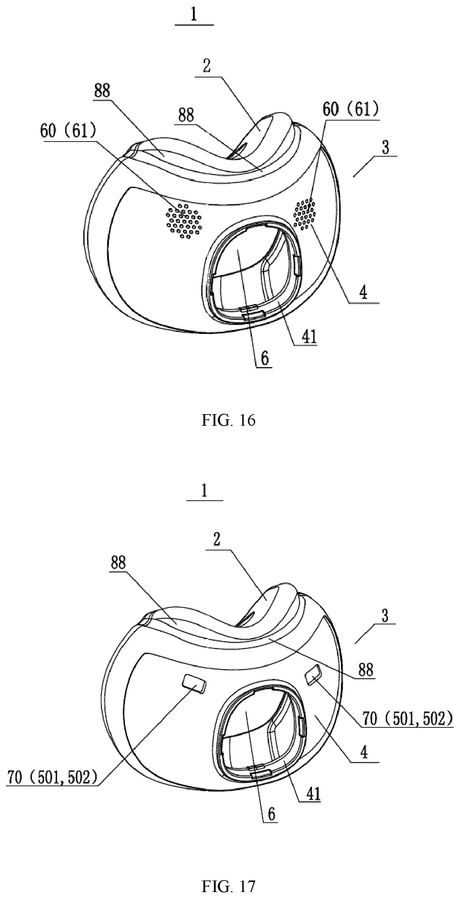

Please refer to FIG. 16, the difference between this embodiment and the foregoing embodiments lies in that the oronasal cushion 1 is configured with exhaust components 60 on the upper front side thereof. The exhaust component 60 includes a plurality of exhaust holes 61 provided on the oronasal cushion 1.

The exhaust hole 61 is in the shape of a strip, an oblong, a circle, an oval or a special shape, etc. Preferably, the exhaust hole 61 is in a long strip shape, which can have a larger area per hole on a narrower surface in the exhaust direction, so that a minimum number of holes can be achieved when the total exhaust gas volume is an expected constant value, which can also reduce the noise and blowing effect on your bed partner.

Furthermore, a plurality of exhaust holes 61 may be arranged in a circular arrangement, an elliptical arrangement, an array hole arrangement, or a special-shaped arrangement, etc. It can be understood that the "ellipse" described in the present disclosure is an approximately elliptical structure or an elliptical-like structure, and is not limited to an ellipse formed according to standard formulas and curvature.

In addition, the inner hole area of the exhaust hole 61 is greater or less than the outer hole area of the exhaust hole 61. Since exhaust holes with the same inner and outer diameters will generate greater noise, the inner hole diameter of the exhaust hole 61 is set to be different from the outer hole diameter of the exhaust hole 61, so as to significantly reduce the exhaust noise.

In addition, the exhaust holes 61 are arranged in a divergent manner, and the exhaust holes 61 are arranged as diffusely as possible. The number of adjacent exhaust holes 61 for each exhaust hole 61 is at most 6.

As shown in FIG. 16, the exhaust holes are provided on both sides of the air inlet 41 and far away from the air inlet 41.

Optionally, the exhaust holes 61 are realized through multiple slides on the mold, or through later laser drilling. However, these solutions are costly, cannot guarantee the quality of the holes, and have high noises.

Preferably, the exhaust hole 61 is formed by kissing off through the mold. The exhaust hole 61 formed by kissing off through the mold has better process formability and lower process cost, can ensure the quality of the hole, and has lower noise during use.

### Embodiment 4

Based on the above embodiments, the present disclosure provides a modified embodiment, that is, Embodiment 4. Only the differences from the foregoing embodiments will be described below, and the similarities will not be repeated.

Please refer to FIG.17, the difference between this embodiment and the foregoing embodiments lies in that the oronasal cushion 1 is configured with ventilation components. The oronasal cushion includes a second body, and a ventilation component 70 (or can be called a safety member) is provided on the second body. The second main body may be integrally formed with the oronasal cushion 1.

The ventilation component 70 is disposed on both sides of the air inlet 41 and far away from the air inlet 41. The ventilation component 70 includes a safety valve port 501 and a safety valve disc 502 provided on the upper front side of the oronasal cushion 1. When the pressurized gas is supplied into the cavity 6 of the oronasal cushion 1 by a pressure device (not shown) through the air inlet 41, the safety valve disc 502 closes the safety valve port 501, so that the pressurized gas is supplied to the wearer through the oronasal cushion 1. On the contrary, when no pressurized gas is supplied into the oronasal cushion 1, the safety valve port 501 is opened by the safety valve disc 502, so that the oronasal cushion 1 is communicated with the atmosphere through the safety valve port, and the wearer can breathe the air outside the oronasal cushion through the safety valve port 501, avoiding the risk of strangulation.

The implement of opening the safety valve port 501 by the safety valve disc 502 can be achieved by using an existing safety valve disc to open the safety valve port, which will not be described in detail in the present disclosure.

### Embodiment 5

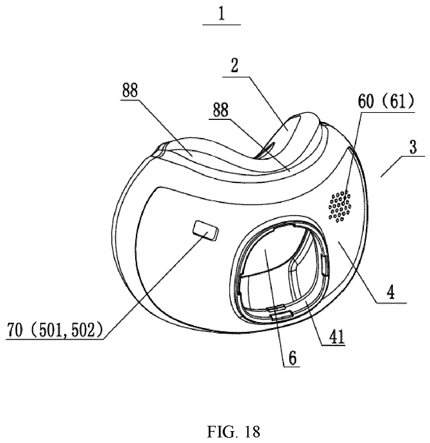

Please refer to FIG.18, based on various embodiments described above, the present disclosure provides a modified embodiment, that is, Embodiment 5. Only the differences from the foregoing embodiments will be described below, and the similarities will not be repeated.

Please refer to FIG.18, the difference between this embodiment and the foregoing embodiments lies in that the oronasal cushion 1 is provided with both the exhaust components 60 (exhaust holes 61) and the ventilation components 70.

The specific arrangement manner of the exhaust holes 61 and the ventilation components 70 may adopt the arrangement manner in Embodiment 3 and Embodiment 4, and will not be repeated here.

According to a second aspect of the present disclosure, the present disclosure also provides a patient interface device, including: a nasal mask, the above-mentioned oronasal cushion 1 provided in the nasal mask, and a frame connected to the oronasal cushion 1. The frame is provided with a headband for fixing with the head of the wearer.

A flexible pipe is connected between the frame and the pressure device, through which the pressurized gas is delivered into the cavity of the nasal mask, and then enters the respiratory tract of the wearer through the nasal opening 21 and the oral opening 31.

## Claims

1. An oronasal cushion (1), wherein the oronasal cushion (1) comprises a nasal structure (2) and an oral structure (3) connected with each other, wherein a penetrating cavity (6) is formed inside the nasal structure (2) and the oral structure (3), and the cavity (6) is configured to receive pressurized gas; the nasal structure (2) is adapted to fit around nostrils (N1) of a wearer for sealing, and the oral structure (3) is adapted to fit around a mouth of the wearer for sealing;
wherein the nasal structure (2) is configured so that no clamping effect is applied on ala nasi (N2) of the wearer when the nasal structure (2) is pressed against a bottom of a nose of the wearer for sealing by the pressurized gas;
and wherein
the oral structure (3) is configured to accommodate the mouth of the wearer and fit around the mouth of the wearer in response to the oronasal cushion (1) being put on by the wearer; and the oral structure (3) comprises an oral soft pad part (32) in contact with a face of the wearer and a reinforcing structure (4) connected with the oral soft pad part (32), and rigidity of the reinforcing structure (4) is greater than rigidity of the oral soft pad part (32);
**characterized in that** the nasal structure (2) comprises a nasal opening (21) in communication with the cavity (6) and a nasal soft pad part (22) surrounding the nasal opening (21), the nasal opening (21) is configured to surround a lower side of the nostrils (N1) of the wearer in response to the oronasal cushion (1) being put on by the wearer, and the nasal soft pad part (22) is adapted to fit around the nostrils (N1) of the wearer for sealing.

2. The oronasal cushion (1) according to claim 1, **characterized in that** the nasal soft pad part (22) comprises a soft intermediate part (23) disposed around the nasal opening (21), the intermediate part (23) is configured to accommodate the nose of the wearer without submerging a nasal apex (N3) of the wearer, and the intermediate part (23) deforms in response to an increase in the pressure in the cavity (6), to fit around the nostrils (N1) of the wearer.

3. The oronasal cushion (1) according to claim 2, **characterized in that** the nasal soft pad part (22) further comprises a first lateral support part and a second lateral support part connected to the intermediate part (23), respectively;
wherein the first lateral support part and the second lateral support part extend on both sides of the intermediate part (23), respectively; or
the first lateral support part and the second lateral support part extend on both sides of the intermediate part (23) respectively until reaching an outer front end of the intermediate part (23).

4. The oronasal cushion (1) according to claim 3, **characterized in that** the intermediate part (23) is configured to be further away from an upper end of the oronasal cushion (1) than the first lateral support part and the second lateral support part.

5. The oronasal cushion (1) according to claim 3 or 4, **characterized in that** rigidity of both the first lateral support part and the second lateral support part is greater than rigidity of the intermediate part (23).

6. The oronasal cushion (1) according to claim 3 or **4, characterized in that** a thickness of the first lateral support part and a thickness of the second lateral support part are greater than a thickness of the intermediate part (23).

7. The oronasal cushion (1) according to any one of claims 2 to **4, characterized in that** the intermediate part (23) is a structure with uniform thickness.

8. The oronasal cushion (1) according to any one of claims 2 to **4, characterized in that** the intermediate part (23) comprises one or more local thickened part (25);
preferably, the local thickened part (25) is provided at a peripheral edge of the intermediate part (23).

9. The oronasal cushion (1) according to claim 3 or **4,** further comprising an oronasal transition part (5) disposed between a rear side of the intermediate part (23) and the oral structure (3), the oronasal transition part (5) is configured to fit with an upper lip region of the wearer in response to the oronasal cushion (1) being put on by the wearer; and
a thickness of the oronasal transition part (5) is the same as a thickness of the intermediate part (23).

10. The oronasal cushion (1) according to claim 9, **characterized in that** the oronasal transition part (5) is configured as a concave structure recessed in a direction away from upper lip of the wearer.

11. The oronasal cushion (1) according to claim 9, **characterized in that** two sides of the oronasal transition part (5) are respectively connected to the first lateral support part and the second lateral support part by recessing in a direction away from upper lip of the wearer.

12. The oronasal cushion (1) according to claim 3 or 4, further comprising an oronasal connection part provided between the nasal structure (2) and the oral structure (3), and a thickness of the oronasal connection part is variable.

13. The oronasal cushion (1) according to claim 12, **characterized in that** the oronasal connection part comprises a first oronasal connection part located between the first lateral support part and a side part of the oral structure (3), a second oronasal connection part located between the second lateral part and the side part of the oral structure (3) and a third oronasal connection part located between a front side of the nasal structure (2) and a front side of the oral structure (3); and
a thickness of the first oronasal connection part and a thickness of the second oronasal connection part are both greater than a thickness of the third oronasal connection part.

14. A patient interface device, **characterized in that** the patient interface device comprises the oronasal cushion (1) according to any one of claims 1 to 13, wherein the patient interface device further comprises a frame, and the oronasal cushion (1) is sealingly connected to the frame.

## Patentansprüche

1. Orinasales Polster (1), wobei das orinasale Polster (1) eine Nasal-Struktur (2) sowie eine Oral-Struktur (3) umfasst, die miteinander verbunden sind, wobei ein durchgehender Hohlraum (6) im Inneren der Nasal-Struktur (2) und der Oral-Struktur (3) ausgebildet ist, und der Hohlraum (6) zum Aufnehmen von Druckgas ausgeführt ist; wobei die Nasal-Struktur (2) so eingerichtet ist, dass sie zum Abdichten um Nasenlöcher (N1) eines Trägers herum passt, und die Oral-Struktur (3) so eingerichtet ist, dass sie zum Abdichten um einen Mund des Trägers herum passt;
wobei die Nasal-Struktur (2) so ausgeführt ist, dass durch das Druckgas keine Klemmwirkung auf Nasenflügel (N2) des Trägers ausgeübt wird, wenn die Nasal-Struktur (2) zum Abdichten auf eine Unterseite einer Nase des Trägers gepresst wird;
und wobei die Oral-Struktur (3) so ausgeführt ist, dass sie in Reaktion darauf, dass das orinasale Polster (1) von dem Träger aufgesetzt wird, den Mund des Trägers aufnimmt und um den Mund des Trägers herum passt; und die Oral-Struktur (3) einen weichen Oral-Auflageteil (32), der in Kontakt mit einem Gesicht des Trägers ist, sowie eine verstärkende Struktur (4) umfasst, die mit dem weichen Oral-Auflageteil (32) verbunden ist, und Steifigkeit der verstärkenden Struktur (4) größer ist als Steifigkeit des weichen Oral-Auflageteils (32);
**dadurch gekennzeichnet, dass** die Nasal-Struktur (2) eine Nasal-Öffnung (21), die in Verbindung mit dem Hohlraum (6) steht, sowie einen weichen Nasal-Auflageteil (22) umfasst, der die Nasal-Öffnung (21) umschließt, die Nasal-Öffnung (21) so ausgeführt ist, dass sie eine untere Seite der Nasenlöcher (N1) des Trägers in Reaktion darauf umschließt, dass das orinasale Polster (1) von dem Träger aufgesetzt wird, und der weiche Nasal-Auflageteil (22) so eingerichtet ist, dass er zum Abdichten um die Nasenlöcher (N1) des Trägers herum passt.

2. Orinasales Polster (1) nach Anspruch 1, **dadurch gekennzeichnet, dass** der weiche Nasal-Auflageteil (22) einen weichen Zwischenteil (23) umfasst, der um die Nasal-Öffnung (21) herum angeordnet ist, wobei der Zwischenteil (23) so ausgeführt ist, dass er die Nase des Trägers aufnimmt, ohne eine Nasenspitze (N3) des Trägers zu überragen, und der Zwischenteil (23) sich in Reaktion auf eine Zunahme des Drucks in dem Hohlraum (6) so verformt, dass er um die Nasenlöcher (N1) des Trägers herum passt.

3. Orinasales Polster (1) nach Anspruch 2, **dadurch gekennzeichnet, dass** der weiche Nasal-Auflageteil (22) des Weiteren einen ersten seitlichen Stützteil sowie einen zweiten seitlichen Stützteil umfasst, die jeweils mit dem Zwischenteil (23) verbunden sind;
wobei sich der erste seitliche Stützteil und der zweite seitliche Stützteil jeweils auf beiden Seiten des Zwischenteils (23) erstrecken; oder
sich der erste seitliche Stützteil und der zweite seitliche Stützteil auf beiden Seiten des Zwischenteils (23) jeweils so weit erstrecken, dass sie bis zu einem äußeren vorderen Ende des Zwischenteils (23) reichen.

4. Orinasales Polster (1) nach Anspruch 3, **dadurch gekennzeichnet, dass** der Zwischenteil (23) so ausgeführt ist, dass er weiter von einem oberen Ende des orinasalen Polsters (1) entfernt ist als der erste seitliche Stützteil und der zweite seitliche Stützteil.

5. Orinasales Polster (1) nach Anspruch 3 oder 4, **dadurch gekennzeichnet, dass** Steifigkeit sowohl des ersten seitlichen Stützteils als auch des zweiten seitlichen Stützteils größer ist als Steifigkeit des Zwischenteils (23).

6. Orinasales Polster (1) nach Anspruch 3 oder 4, **dadurch gekennzeichnet, dass** eine Dicke des ersten seitlichen Stützteils und eine Dicke des zweiten seitlichen Stützteils größer sind als eine Dicke des Zwischenteils (23).

7. Orinasales Polster (1) nach einem der Ansprüche 2 bis 4, **dadurch gekennzeichnet, dass** der Zwischenteil (23) eine Struktur mit einheitlicher Dicke ist.

8. Orinasales Polster (1) nach einem der Ansprüche 2 bis 4, **dadurch gekennzeichnet, dass** der Zwischenteil (23) einen oder mehrere lokal verdickten/verdickte Teil/e (25) umfasst;
wobei sich der lokale verdickte Teil (25) vorzugsweise an einem Umfangsrand des Zwischenteils (23) befindet.

9. Orinasales Polster (1) nach Anspruch 3 oder 4, das des Weiteren einen orinasalen Übergangsteil (5) umfasst, der zwischen einer hinteren Seite des Zwischenteils (23) und der Oral-Struktur (3) angeordnet ist, wobei der orinasale Übergangsteil (5) so ausgeführt ist, dass er in Reaktion darauf, dass das orinasale Polster (1) von dem Träger aufgesetzt wird, an einen Oberlippenbereich des Trägers passt; und
eine Dicke des orinasalen Übergangsteils (5) genauso groß ist wie eine Dicke des Zwischenteils (23).

10. Orinasales Polster (1) nach Anspruch 9, **dadurch gekennzeichnet, dass** der orinasale Übergangsteil (5) als eine konkave Struktur ausgeführt ist, die in einer Richtung von der Oberlippe des Trägers weg vertieft ist.

11. Orinasales Polster (1) nach Anspruch 9, **dadurch gekennzeichnet, dass** 2 Seiten des orinasalen Übergangsteils (5) jeweils mit dem ersten seitlichen Stützteil und dem zweiten seitlichen Stützteil verbunden werden, indem sie in einer Richtung von der Oberlippe des Trägers weg vertieft werden.

12. Orinasales Polster (1) nach Anspruch 3 oder 4, das des Weiteren einen orinasalen Verbindungsteil umfasst, der sich zwischen der Nasal-Struktur (2) und der Oral-Struktur (3) befindet, und wobei eine Dicke des orinasalen Verbindungsteils variabel ist.

13. Orinasales Polster (1) nach Anspruch 12, **dadurch gekennzeichnet, dass** der orinasale Verbindungsteil einen ersten orinasalen Verbindungsteil, der zwischen dem ersten seitlichen Stützteil und einem Seitenteil der Oral-Struktur (3) angeordnet ist, einen zweiten orinasalen Verbindungsteil, der zwischen dem zweiten seitlichen Stützteil und dem Seitenteil der Oral-Struktur (3) angeordnet ist, sowie einen dritten orinasalen Verbindungsteil umfasst, der zwischen einer Vorderseite der Nasal-Struktur (2) und einer Vorderseite der Oral-Struktur (3) angeordnet ist; und
eine Dicke des ersten orinasalen Verbindungsteils und eine Dicke des zweiten orinasalen Verbindungsteils beide größer sind als eine Dicke des dritten orinasalen Verbindungsteils.

14. Patienten-Schnittstellenvorrichtung, **dadurch gekennzeichnet, dass** die Patienten-Schnittstellenvorrichtung das orinasale Polster (1) nach einem der Ansprüche 1 bis 13 umfasst, wobei die Patienten-Schnittstellenvorrichtung des Weiteren einen Rahmen umfasst und das orinasale Polster (1) dichtend mit dem Rahmen verbunden ist.

## Revendications

1. Coussin oronasal (1), dans lequel le coussin oronasal (1) comprend une structure nasale (2) et une structure orale (3) reliées l'une à l'autre, dans lequel une cavité pénétrante (6) est formée à l'intérieur de la structure nasale (2) et de la structure orale (3), et la cavité (6) est configurée pour recevoir du gaz pressurisé ; la structure nasale (2) est adaptée pour s'ajuster autour des narines (N1) d'un porteur pour une fermeture étanche, et la structure orale (3) est adaptée pour s'ajuster autour d'une bouche du porteur pour une fermeture étanche ;
dans lequel la structure nasale (2) est configurée de sorte qu'aucun effet de serrage ne soit appliqué sur une aile du nez (N2) du porteur lorsque la structure nasale (2) est pressée contre le bas du nez du porteur pour une fermeture étanche par le gaz pressurisé ;
et dans lequel
la structure orale (3) est configurée pour accueillir la bouche du porteur et s'ajuster autour de la bouche du porteur en réponse à la mise en place du coussin oronasal (1) par le porteur ; et la structure orale (3) comprend une partie tampon souple oral (32) en contact avec un visage du porteur et une structure de renfort (4) reliée à la partie tampon souple oral (32), et une rigidité de la structure de renfort (4) est supérieure à une rigidité de la partie tampon souple oral (32) ;
**caractérisé en ce que** la structure nasale (2) comprend une ouverture nasale (21) en communication avec la cavité (6) et une partie tampon souple oral nasal (22) entourant l'ouverture nasale (21), l'ouverture nasale (21) est configurée pour entourer un côté inférieur des narines (N1) du porteur en réponse à la mise en place du coussin oronasal (1) par le porteur, et la partie tampon souple nasal (22) est adaptée pour s'ajuster autour des narines (N1) du porteur pour une fermeture étanche.

2. Coussin oronasal (1) selon la revendication 1, **caractérisé en ce que** la partie tampon souple nasal (22) comprend une partie intermédiaire souple (23) disposée autour de l'ouverture nasale (21), la partie intermédiaire (23) est configurée pour accueillir le nez du porteur sans submerger un sommet nasal (N3) du porteur, et la partie intermédiaire (23) se déforme en réponse à une augmentation de la pression dans la cavité (6), pour s'ajuster autour des narines (N1) du porteur.

3. Coussin oronasal (1) selon la revendication 2, **caractérisé en ce que** la partie tampon souple nasal (22) comprend en outre une première partie latérale de support et une seconde partie latérale de support reliées respectivement à la partie intermédiaire (23) ;
dans lequel la première partie latérale de support et la seconde partie latérale de support s'étendent sur les deux côtés de la partie intermédiaire (23), respectivement ; ou
la première partie latérale de support et la seconde partie latérale de support s'étendent respectivement sur les deux côtés de la partie intermédiaire (23) jusqu'à atteindre une extrémité frontale extérieure de la partie intermédiaire (23).

4. Coussin oronasal (1) selon la revendication 3, **caractérisé en ce que** la partie intermédiaire (23) est configurée pour être plus éloignée d'une extrémité supérieure du coussin oronasal (1) que la première partie latérale de support et la seconde partie latérale de support.

5. Coussin oronasal (1) selon la revendication 3 ou 4, **caractérisé en ce que** la rigidité à la fois de la première partie latérale de support et de la seconde partie latérale de support est supérieure à la rigidité de la partie intermédiaire (23).

6. Coussin oronasal (1) selon le revendication 3 ou 4, **caractérisé en ce qu'**une épaisseur de la première partie latérale de support et une épaisseur de la seconde partie latérale de support sont supérieures à une épaisseur de la partie intermédiaire (23).

7. Coussin oronasal (1) selon l'une quelconque des revendications 2 à 4, **caractérisé en ce que** la partie intermédiaire (23) est une structure ayant une épaisseur uniforme.

8. Coussin oronasal (1) selon l'une quelconque des revendications 2 à 4, **caractérisé en ce que** la partie intermédiaire (23) comprend une ou plusieurs parties épaissies locales (25) ;
de préférence, la partie épaissie locale (25) est agencée au niveau d'un bord périphérique de la partie intermédiaire (23).

9. Coussin oronasal (1) selon le revendication 3 ou 4, comprenant en outre une partie de transition oronasale (5) disposée entre un côté arrière de la partie intermédiaire (23) et la structure orale (3), la partie de transition oronasale (5) est configurée pour s'ajuster avec une région de lèvre supérieure du porteur en réponse à la mise en place du coussin oronasal (1) par le porteur ; et
une épaisseur de la partie de transition oronasale (5) est la même qu'une épaisseur de la partie intermédiaire (23).

10. Coussin oronasal (1) selon revendication 9, **caractérisé en ce que** la partie de transition oronasale (5) est configurée sous forme d'une structure concave évidée dans une direction à l'opposé de la lèvre supérieure du porteur.

11. Coussin oronasal (1) selon revendication 9, **caractérisé en ce que** les deux côtés de la partie de transition oronasale (5) sont respectivement reliés à la première partie latérale de support et à la seconde partie latérale de support par évidement dans une direction à l'opposé de la lèvre supérieure du porteur.

12. Coussin oronasal (1) selon le revendication 3 ou 4, comprenant en outre une partie de liaison oronasale agencée entre la structure nasale (2) et la structure orale (3), et une épaisseur de la partie de liaison oranasale est variable.

13. Coussin oronasal (1) selon revendication 12, **caractérisé en ce que** la partie de liaison oronasale comprend une première partie de liaison oronasale située entre la première partie latérale de support et une partie de côté de la structure orale (3), une deuxième partie de liaison oronasale située entre la seconde partie latérale et la partie de côté de la structure orale (3), et une troisième partie de liaison oronasale située entre un côté frontal de la structure nasale (2) et un côté frontal de la structure orale (3) ; et
une épaisseur de la première partie de liaison oronasale et une épaisseur de la deuxième partie de liaison oronasale sont toutes deux supérieures à une épaisseur de la troisième partie de liaison oronasale.

14. Dispositif d'interface patient, **caractérisé en ce que** le dispositif d'interface patient comprend le coussin oronasal (1) selon l'une quelconque des revendications 1 à 13, dans lequel le dispositif d'interface patient comprend en outre un cadre, et le coussin oronasal (1) est relié de manière étanche au cadre.
